(19) Europäisches Patentamt
European Patent Office
Office européen des brevets

(11) Veröffentlichungsnummer : **0 401 591 B1**

(12) **EUROPÄISCHE PATENTSCHRIFT**

(45) Veröffentlichungstag der Patentschrift : **03.03.93 Patentblatt 93/09**

(51) Int. Cl.$^5$ : **A61L 15/07,** C08K 3/34

(21) Anmeldenummer : **90109738.6**

(22) Anmeldetag : **22.05.90**

# (54) Stützverbände mit stark verringerter Schaumbildung.

(30) Priorität : **03.06.89 DE 3918177**

(43) Veröffentlichungstag der Anmeldung : **12.12.90 Patentblatt 90/50**

(45) Bekanntmachung des Hinweises auf die Patenterteilung : **03.03.93 Patentblatt 93/09**

(84) Benannte Vertragsstaaten : **AT BE CH DE DK ES FR GB GR IT LI LU NL SE**

(56) Entgegenhaltungen : **EP-A- 0 259 492**

(73) Patentinhaber : **BAYER AG**
**W-5090 Leverkusen 1 Bayerwerk (DE)**

(72) Erfinder : **Jansen, Bernhard, Dr.**
**Roggendorfstrasse 65**
**W-5000 Köln 80 (DE)**
Erfinder : **Müller, Hanns-Peter, Dr.**
**Weizenfeld 36**
**W-5060 Bergisch-Gladbach (DE)**
Erfinder : **Richter, Roland, Dr.**
**Hermann-Ehlert-Strasse 12**
**W-5090 Leverkusen (DE)**

EP 0 401 591 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Stützverbände mit stark verringerter Schaumbildung

Die Erfindung betrifft Stützverbände, die eine neuartige Polyisocyanat-Zubereitung auf einem Trägermaterial enthalten, ein Verfahren zu ihrer Herstellung und die Verwendung der Polyisocyanat-Zubereitungen auf einem Trägermaterial für Stützverbände.

Die Verwendung von mit Gips imprägnierten Binden als versteifendes Verbandsmaterial ist bekannt. Derartige Gipsverbände sind unerwünscht schwer, wenig luftdurchlässig, verlieren in feuchtem Zustand, beispielsweise durch Einwirkung von Wasser auf den ausgehärteten Verband, rasch an Festigkeit, sie behindern wegen ihrer Röntgenabsorption- und -streuung die diagnostische Auswertung von Röntgenaufnahmen und sind wegen ihrer mangelhaften Wasserfestigkeit oft Anlaß zu Hautirritationen, hervorgerufen durch Bakterien- oder Pilzbewuchs im Verband.

In der DE-PS 2 357 931 werden Stützverbände beschrieben, deren Härtungsprinzip die Reaktion zwischen Isocyanatgruppen und Wassermolekülen ist. Die Verbandsmaterialien bestehen aus einem flexiblen Trägermaterial, welches mit einer Isocyanatgruppen aufweisenden Verbindung, vorzugsweise ein Isocyanat-Prepolymer, imprägniert und/oder beschichtet ist. Hier werden jedoch keinerlei Substanzen verwendet, die die Schaumbildung verhindern sollen.

Nach DE-PS 2 651 089 setzt man als härtbare Komponente des Stützverbandes ein Prepolymer mit aromatischen Isocyanatgruppen ein, welches eine bestimmte Menge an Stickstoff enthält. Nach der EP-PS 86 621 beschleunigt man die Härtungsreaktion des Isocyanat-Prepolymeren mit Bismorpholinodiethylether, einem Katalysator, der die Lagerstabilität des Prepolymeren wenig beeinträchtigt.

In beiden Anmeldungen werden Polydimethylsiloxane, einmal geringer, einmal hoher Viskosität als oberflächenaktive Substanzen zur Verhinderung starken Schäumens erwähnt.

Allen Polyisocyanat-Zubereitungen für Stützverbände ist die Schaumentwicklung gemeinsam, die auf Grund der Kohlendioxidentwicklung in der Isocyanat-Wasser-Reaktion nicht verwunderlich ist.

Die Schaumentwicklung ist jedoch höchst unerwünscht. Hier dafür einige Gründe:

- übermäßige Schaumentwicklung ergibt einen schlechten Lagenverbund des ausgehärteten Verbandes aufgrund von Gasblasen;
- Schaumentwicklung zwingt zur Reduzierung der Menge an Polyisocyanat-Zubereitung pro Gewichtseinheit Trägermaterial und läßt es nicht zu, eine hohe Stabilität des ausgehärteten Verbandes zu erreichen.
- selbst geringe Schaumentwicklung verklebt die durch das Gewebe vorgegebenen Durchlässe im ausgehärteten Verband und macht damit die Luftdurchlässigkeit, einen der größten Vorteile des Polyisocyanat-Stützverbandes, zunichte.

Es ist klar ersichtlich, daß der Schlüssel zu einem verbesserten Polyurethan-Stützverband in der Vermeidung der Schaumentwicklung liegt.

Aufgabe der vorliegenden Anmeldung ist es folglich, diese zuvor genannten Nachteile zu beseitigen. Die Polydimethylsiloxane des Standes der Technik sind jedoch häufig wegen ihres ausgeprägt hydrophoben Charakters mit den Polyisocyanat-Zubereitungen nicht gut mischbar, besonders wenn es sich um solche höheren Molekulargewichte handelt.

Gegenstand der vorliegenden Erfindung sind Stützverbände, die auf einem flexiblen Trägermaterial eine Polyisocyanat-Zubereitung enthalten, bestehend aus:

a) mindestens einem organischen Polyisocyanat,

b) mindestens einem Katalysator und

c) Hilfs- und Zusatzmitteln, dadurch gekennzeichnet, daß als Hilfs- und Zusatzmittel Verbindungen der Struktur I

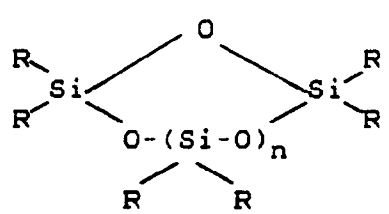

mit R=Methyl, Ethyl, Propyl und $1 \leqq n \leqq 4$, bevorzugt jedoch die Verbindung

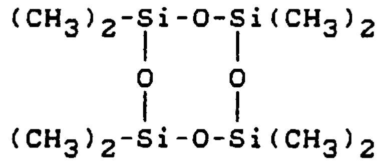

mit Namen Octamethyl-cyclotetrasiloxan verwendet wird.

Die erfindungsgemäßen Stützverbände zeichnen sich dadurch aus, daß die ausgehärteten Verbände sogar bei einer Menge an Polyisocyanat-Zubereitung von 70-90 % des Trägermaterialgewichtes überhaupt kein Aufschäumen zeigen.

Außerdem ermöglicht der Einsatz der erfindungsgemäßen Polyisocyanat-Zubereitungen einen höheren Beharzungsgrad des Trägermaterials. Dies ist auch für technische Anwendungen von großer Bedeutung, da ohne die Anwendung weiterer Hilfsmittel sofort eine optisch einwandfreie Oberfläche des Gegenstandes bei gleichzeitig hoher Festigkeit erhalten wird.

Besonders überraschend für den Fachmann ist die Tatsache, daß die Verbindungen der Struktur I trotz ihres niedermolekularen cyclischen Charakters bessere Entschäumungswirkung zeigt als die höhermolekularen Polydimethylsiloxane aus der Klasse der oberflächenaktiven Substanzen. Der niedermolekulare Charakter der Substanz zeigt sich vielmehr als großer Vorteil, da das Vermischen mit einer Isocyanat-Zubereitung wesentlich erleichtert ist und zudem keine Gefahr der Entmischung oder Phasentrennung besteht.

Die Komponente a) der erfindungsgemäßen Polyisocyanat-Zubereitung besteht aus mindestens einem organischen Polyisocyanat, d.h. aus beliebigen Verbindungen bzw. Gemischen von Verbindungen, die pro Molekül mindestens zwei organisch gebundene Isocyanatgruppen aufweisen.

Hierzu gehören sowohl niedermolekulare Polyisocyanate mit einem unter 400 liegenden Molekulargewicht als auch Modifizierungsprodukte derartiger niedermolekularer Polyisocyanate mit einem aus der Funktionalität und dem Gehalt an funktionellen Gruppen berechenbaren, zwischen 400 und 10.000, vorzugsweise 600 und 8.000 und insbesondere 800 bis 5.000, liegenden Molekulargewicht. Geeignete niedermolekulare Polyisocyanate sind beispielsweise solche der Formel

$$Q\,(NCO)_n, \qquad (II)$$

in der

n = 2 bis 4, vorzugsweise 2,

und

Q einen aliphatischen Kohlenwasserstoffrest mit 2 bis 18, vorzugsweise 6 bis 10 C-Atomen,
einen cycloaliphatischen Kohlenwasserstoffrest mit 4 bis 15, vorzugsweise 5 bis 10 C-Atomen,
einen aromatischen Kohlenwasserstoffrest mit 6 bis 15, vorzugsweise 6 bis 13 C-Atomen,
oder einen araliphatischen Kohlenwasserstoffrest mit 8 bis 15, vorzugsweise 8 bis 13 C-Atomen,

bedeuten.

Geeignete derartige niedermolekulare Polyisocyanate sind z.B. Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethylcyclohexan, Hexahydro-1,3- und/oder -1,4-phenylendiisocyanat, Perhydro-2,4′- und/oder -4,4′-diphenylmethan-diisocyanat, 1,3- und 1,4-Phenylendiisocyanat, 2,4- und 2,6-Toluylen-diisocyanat sowie beliebige Gemische dieser Isomeren, Diphenylmethan-2,4′- und/oder -4,4′-diisocyanat, Naphthylen-1,5-diisocyanat, Triphenylmethan-4,4′, 4″-triisocyanat oder Polyphenyl-polymethylenpolyisocyanate, wie sie durch Anilin-Formaldehyd-Kondensation und anschließende Phosgenierung erhalten werden.

Geeignete höhermolekulare Polyisocyanate sind Modifizierungsprodukte derartiger einfacher Polyisocyanate, d.h. Polyisocyanate mit z.B. Isocyanurat-, Carbodiimid-, Allophanat-, Biuret- oder Uretdion-Strukturheinheiten, wie sie nach an sich bekannten Verfahren des Standes der Technik aus den beispielhaft genannten einfachen Polyisocyanaten der oben genannten allgemeinen Formel hergestellt werden können. Unter den höhermolekularen, modifizierten Polyisocyanaten sind insbesondere die aus der Polyurethanchemie bekannten Prepolymeren mit endständigen Isocyanatgruppen des Molekulargewichtsbereichs 400 bis 10.000, vorzugsweise 600 bis 8.000 und insbesondere 800 bis 5.000 von Interesse. Diese Verbindungen werden in an sich bekannter Weise durch Umsetzung von überschüssigen Mengen an einfachen Polyisocyanaten der beispielhaft genannten Art mit organischen Verbindungen mit mindestens zwei gegenüber Isocyanatgruppen reaktionsfähigen Gruppen, insbesondere organischen Polyhydroxylverbindungen hergestellt. Geeignete derartige Polyhydroxylverbindungen sind sowohl einfache mehrwertige Alkohole des Molekulargewichtsbereichs 62 bis 599, vorzugsweise 62 bis 200 wie z.B. Ethylenglykol, Trimethylolpropan, Propandiol-1,2 oder

Butandiol-1,2, insbesondere jedoch höhermolekulare Polyetherpolyole und/oder Polyesterpolyole der aus der Polyurethanchemie an sich bekannten Art mit Molekulargewichten von 600 bis 8.000, vorzugsweise 800 bis 4.000, die mindestens zwei, in der Regel 2 bis 8, vorzugsweise aber 2 bis 4 primäre und/oder sekundäre Hydroxylgruppen aufweisen. Selbstverständlich können auch solche NCO-Prepolymere eingesetzt werden, die beispielsweise aus niedermolekularen Polyisocyanaten der beispielhaft genannten Art und weniger bevorzugten Verbindungen mit gegenüber Isocyanatgruppen reaktionsfähigen Gruppen wie z.B. Polythioetherpolyolen, Hydroxylgruppen aufweisenden Polyacetalen, Polyhydroxypolycarbonaten, Hydroxylgruppen aufweisenden Polyesteramiden oder Hydroxylgruppen aufweisenden Copolymerisaten olefinisch ungesättigter Verbindungen erhalten worden sind. Zur Herstellung der NCO-Prepolymeren geeignete Verbindungen mit gegenüber Isocyanatgruppen reaktionsfähigen Gruppen, insbesondere Hydroxylgruppen, sind beispielsweise die in US-PS 4 218 543, Kolonne 7, Zeile 29 bis Kolonne 9, Zeile 25 beispielhaft offenbarten Verbindungen. Bei der Herstellung der NCO-Prepolymeren werden diese Verbindungn mit gegenüber Isocyanatgruppen reaktionsfähigen Gruppen mit einfachen Polyisocyanaten der oben beispielhaft genannten Art unter Einhaltung eines NCO/OH-Äquivalentverhältnissses von ca. 1,5:1 bis 20:1, vorzugsweise 5:1 bis 15:1 zur Umsetzung gebracht. Die NCO-Prepolymeren weisen im allgemeinen einen NCO-Gehalt von 2,5 bis 25, vorzugsweise 6 bis 22 Gew.-% auf. Hieraus geht bereits hervor, daß im Rahmen der vorliegenden Erfindung unter "NCO-Prepolymeren" bzw. unter "Prepolymeren mit endständigen Isocyanatgruppen" sowohl die Umsetzungsprodukte als solche als auch ihre Gemische mit überschüssigen Mengen an nicht umgesetzten Ausgangspolyisocyanaten, die oft auch als "Semiprepolymere" bezeichnet werden, zu verstehen sind.

Für das erfindungsgemäße Verfahren besonders bevorzugte Polyisocyanatkomponenten a) sind die in der Polyurethanchemie üblichen technischen Polyisocyanate, d.h. Hexamethylendiisocyanat, 1-Isocyanato-3,3,5-trimethyl-5-isocyanatomethyl-cyclohexan (Isophorondiisocyanat, abgekürzt: IPDI), 4,4′-Diisocyanato-dicyclohexylmethan, 2,4-diisocyanatotoluol, dessen technische Gemische mit 2,6-Diisocyanatotoluol, 4,4′-Diisocyanatodiphenylmethan, dessen Gemische mit den entsprechenden 2,4-′ und 2,2′-Isomeren, Polyisocyanatgemische der Diphenylmethanreihe wie sie durch Phosgenierung von Anilin/Formaldehyd-Kondensaten in an sich bekannter Weise gewonnen werden können, die Biuret- oder Isocyanuratgruppen aufweisenden Modifizierungsprodukte dieser technischen Polyisocyanate und insbesondere NCO-Prepolymere der genannten Art auf Basis dieser technischen Polyisocyanate einerseits und der beispielhaft genannten einfachen Polyolen und-/oder Polyetherpolyolen und/oder Polyesterpolyolen andererseits sowie beliebigen Gemische derartiger Polydiisocyanate.

Bei der Katalysatorkomponente b) handelt es sich um aminische Katalysatoren, bevorzugt jedoch um Bismorpholinodiethylether, wie in EP-PS 86 621 verwendet, oder um Zinnkatalysatoren, bevorzugt jedoch um Dialkylzinndilaurat, blockiert mit Tosylisocyanat wie in der DE-PS 3 326 566 beschrieben.

Gleichfalls katalytisch wirksam und auch bevorzugt sind auf Aminen gestartete Polyether, welche durch einen Gehalt an tert.-Amin von 0,05 bis 10 Gew.-%, bevorzugt aber 0,1 bis 1 Gew.-% (bezogen auf die gesamte Isocyanat-Zubereitung) die Aushärtezeiten der unter a) beschriebenen Prepolymere bestimmen und als Bestandteil des auf Seite 7 erwähnten Prepolymere in die Rezeptur Eingang finden.

Bei den Hilfs- und Zusatzmitteln c) handelt es sich um Octamethyl-cyclotetrasiloxan; die Aufwandsmenge beträgt: 0,01 bis 7 Gew.-%, bevorzugt sind 0,02 bis 5 Gew.-%, besonders bevorzugt sind 0,05 bis 4 Gew.-%.

Über die genannten Komponenten c) hinaus können auch gegebenenfalls die aus der Klebstoff- und Lacktechnologie bekannten Lösungsmittel wie z.B. Toluol, Xylol, Ethylacetat, Butylacetat, Methylethylketon, Methylisobutylketon, Ethylenglykol-monoethyletheracetat oder beliebige Gemische derartiger Lösungsmittel verwendet werden. Weitere Komponenten c) sind beispielsweise Pigmente, Füllstoffe oder Verlaufshilfsmittel sowie UV-Stabilisatoren.

Als Trägermittel kommen massive oder poröse Folien oder auch Schaumstoffe aus natürlichen oder synthetischen Materialien (z.B. Polyurethan) in erster Linie luftdurchlassige, flexible Flächengebilde auf textiler Basis in Betracht, vorzugsweise mit einem Flächengewicht von 20 bis 1.000 g/m$^2$, insbesondere von 30 bis 500 g/m$^2$. Als Flächengebilde seien beispielsweise genannt:

1. Textile Gewebe, Gewirke oder Gestricke eines Flächengewichts von 20 bis 200 g/m$^2$, vorzugsweise 40 bis 100 g/m$^2$ mit einer Fadenzahl von vorzugsweise 2 bis 20 Fäden je laufenden Zentimeter in der Längs- und Querrichtung. Das textile Gewebe oder Gewirke kann aus beliebigen natürlichen oder synthetischen Garnen hergestellt sein. Vorzugsweise werden jedoch Gewebe oder Gewirke eingesetzt, die aus Baumwollgarnen oder aus Mischgarnen hergestellt worden sind, die ihrerseits sowohl aus hydrophoben Fäden bzw. Fasern eines hohen E-Moduls (beispielsweise Polyester) und hydrophilen natürlichen oder synthetischen Fäden bzw. Fasern (beispielsweise Baumwolle oder Polyamid) erhalten worden sind.
2. Glasfasergewebe, -gewirke oder -gestricke eines Flächengewichts von 60 bis 500 g/m$^2$, vorzugsweise 100 bis 400 g/m$^2$, hergestellt aus Glasfasergarnen mit einem E-Modul von 7.000 bis 9.000 (daN/mm$^2$ ), und einer Fadenzahl von 3 bis 10, bevorzugt 5 bis 7 in Längsrichtung und mit einer Fadenzahl von 3 bis

10, bevorzugt 4 bis 6 in Querrichtung je Zentimeter Glasfasergewebe, die durch eine spezielle Art der Hitzebehandlung eine Längenelastizität von 10 bis 30 % besitzen, sind bevorzugt. Die Gewirke können sowohl geschlichtet als auch ungeschlichtet sein.

3. Nicht gebundene oder gebundene oder genadelte Vliese auf Basis von anorganischen und vorzugsweise organischen Fasern eines Flächengewichts von 30 bis 400 g/m², vorzugsweise 50 bis 200 g/m².

Für die Herstellung erfindungsgemäßer Versteifungsverbände in Form von Schalen oder Schienen kommen auch Vliese mit Flächengewichten bis 1.000 g/m² in Betracht. Erfindungsgemäß geeignete Trägermaterialien werden beispielsweise auch in US-PS- 4 134 397, US_PS 3 686 725, US-PS 3 882 857, DE-PS 3 211 634 und EP-PS 61 642 beschrieben.

Bei den erfindungsgemäßen Stützverbänden ist das Trägermaterial mit einer Menge von 25 bis 90 Gew.-%, bevorzugt 25 bis 80 Gew.-%, besonders bevorzugt von 30 bis 75 Gew.-%, bezogen auf den gesamten Stützverband, beschichtet und/oder imprägniert.

Gegenstand der vorliegenden Erfindung ist auch ein Verfahren zur Herstellung von Stützverbänden, das dadurch gekennzeichnet ist, daß bei Feuchtigkeitsausschluß eine Polyisocyanat-Zubereitung bestehend aus

a) mindestens einem organischen Polyisocyanat,

b) mindestens einem Katalysator und

c) Hilfs- und Zusatzmitteln, wobei als erfindungsgemäßes Hilfs- und Zusatzmittel die Verbindungen der Struktur I, bevorzugt aber Octamethyl-cyclotetrasiloxan verwendet wird.

Bei dem erfindungsgemäßen Verfahren arbeitet man unter Ausschluß von Feuchtigkeit. Bevorzugt wird bei einer relativen Feuchte kleiner 1 % (bei 21°C), besonders bevorzugt bei kleiner 0,5 % (bei 21°C), gearbeitet.

Für die Beschichtung bzw. Imprägnierung kann die Polyisocyanat-Zubereitung in einem inerten Lösungsmittel gelöst sein, das nach dem Beschichtungsvorgang wieder abgezogen wird.

Inerte Lösungsmittel können beispielsweise chlorierte Kohlenwasserstoffe, wie Methylenchlorid, Trichlorethan oder Chloroform, Ketone wie Aceton und Methylethylketon sein, Ester wie Essigsäureethylester und Butylacetat, Aromaten wie Toluol, Xylol oder entsprechend derivatisierte Typen, die keinen Zerewitinoff-aktiven Wasserstoff besitzen.

Beispielsweise können die erfindungsgemäßen Stützverbände wie folgt hergestellt werden:

Im allgemeinen läßt man das Trägermaterial über eine Walze laufen und tränkt es mit der Polyisocyanat-Zubereitung gegebenenfalls in einem Lösungsmittel. Unmittelbar nach der Beschichtung bzw. Imprägnierung wird das Verbandsmaterial in der gewünschten Länge (in der Regel 2 bis 11 Meter) auf geeignete Spulen aufgerollt und in einer luft- und wasserdichten Folie (z.B. aus Kunststoff-Aluminiumlaminat) oder anderen völlig dichten Behältern versiegelt, wie es in der DE-OS 2 357 931, DE-OS 2 651 089 und DE-OS 3 033 569 beschrieben wird.

Unmittelbar vor der Anwendung wird das Material aus der Verpackung entnommen und um den zu schützenden Körperteil gewickelt, der gegebenenfalls zuerst mit einem geeigneten Polster- bzw. Unterfütterungsmaterial (z.B. Polyestervlies) umgeben wurde.

Für eine Aushärtung mit Wasser werden beispielsweise Katalysatorkonzentrationen von 0,01 bis 15 Gew.-%, insbesondere bevorzugt von 0,5 bis 12 Gew.-%, verwendet.

Für eine Aushärtung durch Luftfeuchtigkeit werden Katalysatorkonzentrationen von 1,0 bis 15 Gew.-%, bevorzugt 2,0 bis 14 Gew.-%, bevorzugt.

Vorteilhafterweise beginnt die Härtungsreaktion des erfindungsgemäßen Verbandmaterials nicht sofort bei dem Kontakt mit Wasser. Die eigentliche Vernetzungsreaktion zwischen Isocyanatgruppen und Wasser setzt erst nach einer gewissen Zeit ein, die ihrerseits wieder über die Konzentration an Katalysator eingestellt werden kann. Während dieser Anfangsphase des Aushärtens kann der Verband angelegt und modelliert werden.

<u>Beispiel 1</u>

<u>Herstellung eines erfindungsgemäßen wasserhärtenden Reaktivharzes (unter Verwendung des erfindungsgemäßen Additivs)</u>

In einem 10 l Sulfiergefäß mit Edelstahl-Ankerrührer werden 64,0 Teile Isocyanat [Bis-(4-isocyanato-phenyl)-methan], welches carbodiimidisierte Anteile enthält [NCO-Gehalt = 29 %] vorgelegt. Dann werden 0,08 Teile des Hilfs- und Zusatzmittels Octamethylcyclotetrasiloxan und 0,04 Teile Benzoylchlorid sowie danach 19,7 Teile eines durch Propoxylierung von Propylenglykol hergestellten Polyethers (OH-Zahl = 112 mg KOH/g) 13,24 Teile eines durch Propoxylierung von Propylenglykol hergestellten Polyethers (OH-Zahl = 250 mg KOH/g) und 1,7 Teile Dimorpholinodiethylether zugeben.

Der Isocyanatgehalt beträgt am Ende 13,2 %, die Viskosität liegt bei 25.500 mPa.s (23°C).

Beispiel 2

Herstellung eines erfindungsgemäßen wasserhärtenden Reaktivharzes [unter Verwendung eines Additivs des Standes der Technik (Vergleichsbeispiel)]

In einem 10 l Sulfiergefäß mit Edelstahl-Ankerrührer werden 64,0 Teile Isocyanat [Bis-(4-isocyanato-phenyl)-methan, welches carbodiimidisierte Anteile enthält (NCO-Gehalt = 29 %) vorgelegt. Dann werden 0,08 Teile eines Polydimethylsiloxans mit y = 30.000 mPa.s und 0,04 Teile Benzoylchlorid sowie danach 19,7 Teile eines durch Propoxylierung von Propylenglykol hergestellten Polyethers (OH-Zahl = 112 mg KOH/g), 13,24 Teile eines durch Propoxylierung von Propylenglykol hergestellten Polyethers (OH-Zahl = 250 mg KOH/g) und 1,7 Teile Dimorpholinodiethylether zugegeben.

Der Isocyanatgehalt beträgt am Ende 13,2 %, die Viskosität liegt bei 25.500 mPa·s (23°C).

Beispiel 3

Herstellung eines wasserhärtenden Reaktivharzes (Beispiel mit erfindungsgemäßen Additiv)

In einem 10 l Sulfiergefäß mit Edelstahl-Ankerrührer werden 66 Teile Isocyanat (Bis-(4-isocyanatophenyl)-methan), welches carbodiimidisierte Anteile erhält [NCO-Gehalt = 29 %], vorgelegt und auf ca. 50°C vorgewärmt. Dazu werden 1,5 Teile eines UV-Stabilisators (ein Cyanalkylindolderivat) zugegeben und so lange gerührt, bis der gesamte Feststoff gelöst ist. Nach Abkühlen auf Raumtemperatur werden 34 Teile propoxylierten Triethanolamins (OH-Zahl = 150 mg KOH/g) innerhalb 10 Minuten zugesetzt. Nach kurzem Temperaturanstieg auf 55°C nach 55 Minuten fällt die Temperatur wieder, 0,5 Teile des Hilfs- und Zusatzmittels Octamethylcyclotetrasiloxan werden zugegeben und der Isocyanatgehalt erreicht nach 2 Stunden 13,4 %. Der Isocyanatgehalt des fertigen Prepolymers beträgt 12,7 %, die Viskosität liegt bei 14.640 m.Pas (25°C).

Beispiel 4

Herstellung eines wasserhärtenden Reaktivharzes (Vergleichsbeispiel mit einem Additiv des Standes der Technik)

In einem 10 l Sulfiergefäß mit Edelstahl-Ankerrührer werden 66 Teile Isocyanat (Bis-(4-isocyanatophenyl)-methan), welches carbodiimidisierte Anteile erhält [NCO-Gehalt = 29 %], vorgelegt und auf ca 50°C vorgewärmt. Dazu werden 1,5 Teile eines UV-Stabilisators (ein Cyanalkylindolderivat) zugegeben und so lange gerührt, bis der gesamte Feststoff gelöst ist. Nach Abkühlen auf Raumtemperatur werden 34 Teile propoxylierten Triethanolamins (OH-Zahl = 150 mg KOH/g) innerhalb 10 Minuten zugesetzt. Nach kurzem Temperaturanstieg auf 55°C nach 55 Minuten fällt die Temperatur wieder, 0,5 Teile eines Polydimethylsiloxans mit y = 200 mPa.s werden zugegeben, und der Isocyanatgehalt erreicht nach 2 Stunden 13,4 %. Der Isocyanatgehalt des fertigen Prepolymers beträgt 12,7 %, die Viskosität liegt bei 14.640 mPa.s (25°C).

Beispiel 5

Herstellung von Probeverbänden mit den Reaktivharzen der Beispiele 1-4

Beispiel 5a

Ein Glasfasergewirke (Breite 10,0 cm, Quadratmetergewicht ca. 290 g/$m^2$ ), das in Längsrichtung eine Dehnung von ca. 65 % aufweist (eine ausführliche Beschreibung dieser Gewirke findet sich in der US 4 609 578), wird mit 70 Gew.-% (bezogen auf Gewirke) des Harzes gemäß Beispiel 3 beschichtet. Die Beschichtung erfolgt in einer Atmosphäre, deren relative Feuchte durch einen Taupunkt des Wassers von unterhalb -20°C gekennzeichnet ist. Das Harz wird über ein geeignetes Walzenimprägniermittel homogen auf das Gewirke aufgebracht. Eine geeignete Apparatur ist in der US 4 427 002 detailliert beschrieben. Nach Beschichtung werden 3,66 m dieses Bandes auf einer Kunststoffspule von 1 cm Durchmesser aufgewickelt und in einer wasserdampfundurchlässigen Folie versiegelt.

Beispiel 5b

Analog zu Beispiel (5a) wird das Glasfasergewirke mit 70 Gew.-% (bezogen auf Gewirke) des Harzes aus

Beispiel 4 beschichtet und verpackt.

Beispiel 5c

Analog zu Beispiel (5a) wird ein Polyestergewirke (Breite 10,0 cm, Quadratmetergewicht 118 g/m$^2$ ), das in Längsrichtung eine Dehnung von ca. 55 % und in Querrichtung eine Dehnung von ca. 90 % aufweist und welches im Maschenstab ein polyfiles, texturiertes Polyesterfilamentgarn (167 dtex, f 30x1) und in der Maschenreihe eine polyfiles hochfestes Polyesterfilamentgarn (550 dtex, f 96, normal schrumpfend) besitzt, mit 136 Gew.-% (bezogen auf Gewirke) des Harzes aus Beispiel 1 beschichtet und verpackt.

Beispiel 5d

Analog Beispiel (5a) wird das in Beispiel (5c) beschriebene Polyestergewirke mit 136 Gew.-% (bezogen auf Gewirke) des Harzes aus Beispiel 2 beschichtet und verpackt.

Beispiel 5e

Analog Beispiel (5a) wird das in Beispiel (5c) beschriebene Polyestergewirke mit 155 Gew.-% (bezogen auf Gewirke) des Harzes aus Beispiel 1 beschichtet und verpackt.

Beispiel 5f

Analog Beispiel (5a) wird das in Beispiel (5c) beschriebene Polyestergewirke mit 155 Gew.-% (bezogen auf Gewirke) des Harzes aus Beispiel 2 beschichtet und verpackt.

Beispiel 6

Aushärtung der im Beispiel 5 beschriebenen Probeverbände und Ermittlung der Luftdurchlässigkeit der entstandenen Wickelkörper. Durch Eintauchen in Wasser werden die Stützverbände aus den Beispielen 5a bis 5f benetzt und auf einen Zylinder mit einem Außendurchmesser von 7,5 cm aufgewickelt, so daß Wickelkörper mit einem Innendurchmesser von 7,5 cm entstehen.

Nach Aushärtung und vollständiger Trocknung des Probekörpers läßt sich die Luftdurchlässigkeit desselben aus dem Strömungswiderstand hindurchgeblasener Luft ermitteln. Die für die Probeverbände nach Beispiel 5a bis 5f gefundenen Werte sind in der folgenden Tabelle zusammengefaßt.

Tabelle

| Probeverband nach Beispiel | erfindungsgemäßes Additiv | ermittelte Luftdurchlässigkeit in % |
|---|---|---|
| 5a | ja | 82 |
| 5b | nein | 17 |
| 5c | ja | 85 |
| 5d | nein | 50 |
| 5e | ja | 40 |
| 5f | nein | 15 |

Es ist anzumerken, daß das erfindungsgemäße Hilfs- und Zusatzmittel Octamethylcyclotetrasiloxan keine Beeinträchtigung der Lagerstabilität der Isocyanat-Reaktivharze bewirkt.

## Patentansprüche

### Patentansprüche für folgende Vertragsstaaten : AT, BE, CH, DE, DK, FR, GB, GR, IT, LI, LU, NL, SE

1. Stützverbände, die auf einem flexiblen Trägermaterial eine Polyisocyanat-Zubereitung enthalten, bestehend aus:
   a) mindestens einem organischen Polyisocyanat,
   b) mindestens einem Katalysator und
   c) Hilfs- und Zusatzmitteln, dadurch gekennzeichnet, daß die Hilfs- und Zusatzmittel Verbindungen der Struktur I

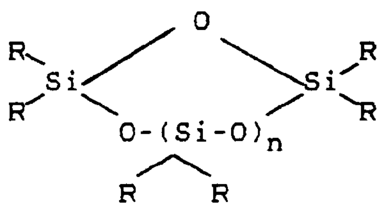

(I)

   in der R für Methyl, Ethyl oder Propyl steht und $1 \leqq n \leqq 4$ bedeutet, entsprechen.

2. Stützverbände nach Anspruch 1, dadurch gekennzeichnet, daß das Hilfs- und Zusatzmittel der Verbindung Octamethylcyclotetrasiloxan der Struktur

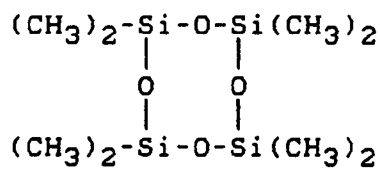

   entspricht.

3. Stützverbände nach den Ansprüchen 1 und 2, dadurch gekennzeichnet, daß die Aufwandsmenge an Hilfs- und Zusatzmittel c) 0,01 bis 7 Gew.-% beträgt.

4. Stützverbände nach den Ansprüchen 1, 2 und 3, dadurch gekennzeichnet, daß sie als organisches Polyisocyanat a) niedermolekulare Polyisocyanate mit einem unter 400 liegenden Molekulargewicht Modifizierungsprodukte derartiger niedermolekularer Polyisocyanate mit einem aus der Funktionalität und dem Gehalt an funktionellen Gruppen berechenbaren, zwischen 400 und 10.000, liegenden Molekulargewicht oder höhermolemulare Polyisocyanate mit z.B. Isocyanurat-, Carbodiimid-, Allophanat-, Biuret- oder Uretdion-Struktureinheiten enthalten.

5. Stützverbände nach den Ansprüchen 1 bis 4, dadurch gekennzeichnet, daß sie als Katalysator b) anionische Katalysatoren oder auf Aminen gestartete Polyether enthalten.

6. Stützverbände nach den Ansprüchen 1 bis 5, dadurch gekennzeichnet, daß sie als Trägermaterial massive oder poröse Folien oder Schaumstoffe aus natürlichen oder synthetischen Materialien oder luftdurchlässige, flexible Flächengebilde auf textiler Basis enthalten.

7. Verfahren zur Herstellung von Stützverbänden nach Anspruch 1, dadurch gekennzeichnet, daß man bei Feuchtigkeitsansluß eine Polyisocyanat-Zubereitung, bestehend aus
   a) mindestens einem organischen Polyisocyanat,
   b) mindestens einem Katalysator und
   c) Hilfs- und Zusatzmitteln der Struktur I gemäß Anspruch 1

   homogen über die Fläche des Trägermaterials verteilt.

8. Verfahren zur Herstellung von Stützverbänden nach Anspruch 7, dadurch gekennzeichnet, daß der Gehalt der Polyisocyanat-Zubereitung 25 bis 90 Gew.-%, bezogen auf den gesamten Stützverband beträgt.

9. Verfahren zur Herstellung von Stützverbänden nach den Ansprüchen 7 und 8, dadurch gekennzeichnet, daß man die Polyisocyanat-Zubereitung in einem inerten Lösungsmittel löst, die Lösung homogen über die Fläche des Trägermaterials verteilt und das Lösungsmittel abzieht.

10. Verwendung von Polyisocyanat-Zubereitungen, bestehend aus
a) mindestens einem organischen Polyisocyanat,
b) mindestens einem Katalysator und
c) Hilfs- und Zusatzmitteln, wobei als erfindungsgemäßes Hilfs- und Zusatzmittel die Verbindungen der Struktur I
gemäß Anspruch 1
zur Herstellung von Stützverbänden für medizinische oder technische Zwecke.

**Patentansprüche für folgenden Vertragsstaat : ES**

1. Verfahren zur Herstellung von Stützverbänden, die auf einem flexiblen Trägermaterial eine Polyisocyanat-Zubereitung enthalten, bestehend aus
a) mindestens einem organischen Polyisocyanat,
b) mindestens einem Katalysator und
c) Hilfs- und Zusatzmitteln, dadurch gekennzeichnet, daß die Hilfs- und Zusatzmittel Verbindungen der Struktur I

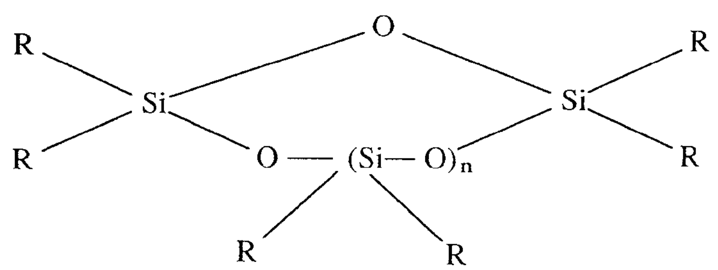

(I)

in der R für Methyl, Ethyl oder Propyl steht und $1 \leqq n \leqq 4$ bedeutet, entsprechen, dadurch gekennzeichnet, daß man bei Feuchtigkeitsanschluß eine Polyisocyanat-Zubereitung, bestehend aus
a) mindestens einem organischen Polyisocyanat,
b) mindestens einem Katalysator und
c) Hilfs- und Zusatzmittel der Struktur I
homogen über die Fläche des Trägermaterials verteilt.

2. Verfahren gemäß Anspruch 1, dadurch gekennzeichnet, daß der Gehalt der Polyisocyanat-Zubereitung 25 bis 90 Gew.-%, bezogen auf den gesamten Stützverband beträgt.

3. Verfahren gemäß Ansprüchen 1 und 2, dadurch gekennzeichnet, daß man die Polyisocyanat-Zubereitung in einem inerten Lösungsmittel löst. die Lösung homogen über die Fläche des Trägermaterials verteilt und das Lösungsmittel abzieht.

**Claims**

**Claims for the following Contracting States : AT, BE, CH, DE, DK, FR, GB, GR, IT, LI, LU, NL, SE**

1. Fixed dressings which contain a polyisocyanate preparation on a flexible backing material, the polyisocyanate preparation being composed of:
a) at least one organic polyisocyanate,
b) at least one catalyst and
c) auxiliaries and additives, characterized in that the auxiliaries and additives used are compounds of the structure I

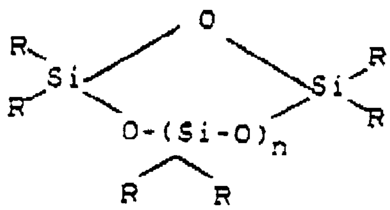

(I)

in which R represents methyl, ethyl or propyl and $1 \leqq n \leqq 4$.

2. Fixed dressings according to claim 1, characterized in that the auxiliary and additive is the compound octamethylcyclotetrasiloxane of the structure

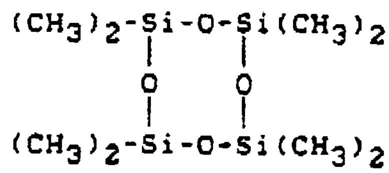

3. Fixed dressings according to claims 1 and 2, characterized in that the amount of auxiliary and additive c) used is 0.01 to 7% by weight.

4. Fixed dressings according to Claims 1, 2 and 3, characterized in that the organic polyisocyanate a) which they contain is a) a low molecular weight polyisocyanate having a molecular weight below 400, a modification of this type of low molecular weight polyisocyanate having a molecular weight, calculable from the functionality and the proportion of functional groups, of between 400 and 10,000 or a higher molecular weight polyisocyanate having, for example, isocyanurate, carbodiimide, allophanate, biuret or uretdione structural units.

5. Fixed dressings according to Claims 1 to 4, characterized in that the catalyst b) which they contain is an anionic catalyst or a polyether which has been polymerized initially in the presence of amines.

6. Fixed dressings according to Claims 1 to 5, characterized in that the backing arterial which they contain is a solid or porous film or foam made from natural or synthetic materials or an air permeable, flexible sheetlike structure based on textiles.

7. Process for the preparation of fixed dressings according to claim 1, characterized in that, with the exclusion of moisture, a polyisocyanate preparation composed of
   a) at least one organic polyisocyanate,
   b) at least one catalyst and
   c) auxiliaries and additives of the structure I according to Claim 1
   is distributed homogeneously over the surface of the backing material.

8. Process for the preparation of fixed dressings according to Claim 7, characterized in that the proportion of polyisocyanate preparation is 25 to 90% by weight, based on the overall fixed dressing.

9. Process for the preparation of fixed dressings according to Claims 7 and 8, characterized in that the polyisocyanate preparation is dissolved in an inert solvent, the solution is distributed homogeneously over the surface of the backing material and the solvent is evaporated off.

10. The use of polyisocyanate preparations composed of
   a) at least one organic polyisocyanate,
   b) at least one catalyst and
   c) auxiliaries and additives, the auxiliaries and additives according to the invention being the compounds of the structure I according to Claim 1 for the preparation of fixed dressings for medical purposes or bandages for engineering purposes.

**Claims for the following Contracting State : ES**

1. Process for the preparation of fixed dressings which contain a polyisocyanate preparation on a flexible backing material, the polyisocyanate preparation being composed of:
   a) at least one organic polyisocyanate,
   b) at least one catalyst and
   c) auxiliaries and additives, characterized in that the auxiliaries and additives used are compounds of the structure I

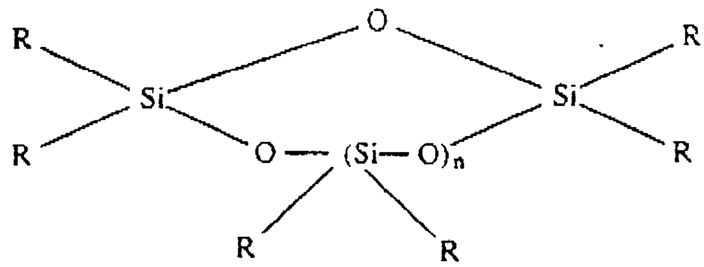

(I)

   in which R represents methyl, ethyl or propyl and $1 \leqq n \leqq 4$,
   characterized in that, with the exclusion of moisture, a polyisocyanate preparation composed of
   a) at least one organic polyisocyanate,
   b) at least one catalyst and
   c) auxiliaries and additives of the structure I is distributed homogeneously over the surface of the backing material.

2. Process according to Claim 1, characterized in that the proportion of polyisocyanate preparation is 25 to 90% by weight, based on the overall fixed dressing.

3. Process according to Claims 1 and 2, characterized in that the polyisocyanate preparation is dissolved in an inert solvent, the solution is distributed homogeneously over the surface of the backing material and the solvent is evaporated off.

**Revendications**

**Revendications pour les Etats contractants suivants : AT, BE, CH, DE, DK, FR, GB, GR, IT, LI, LU, NL, SE**

1. Bandages de soutien qui contiennent, sur une matière de support flexible, une préparation de polyisocyanate composée de:
   a) au moins un polyisocyanate organique,
   b) au moins un catalyseur et
   c) des adjuvants et des additifs, se caractérisant par le fait que les adjuvants et les additifs correspondent à des composés de structure I

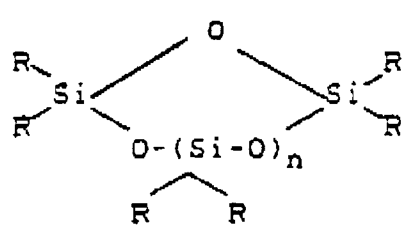

(I)

   dans laquelle R représente un groupe méthyle, éthyle ou propyle et $1 \leqq n \leqq 4$.

2. Bandages de soutien selon la revendication 1, caractérisés en ce que l'adjuvant et l'additif correspondent au composé octaméthylcyclotétrasiloxane de structure

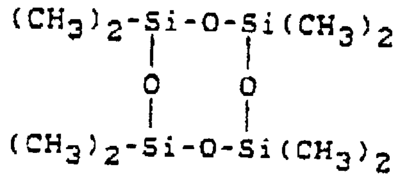

3. Bandages de soutien selon les revendications 1 et 2, caractérisés en ce que la quantité d'application en adjuvant et additif c) varie entre 0,01 et 7% en poids.

4. Bandages de soutien selon les revendications 1, 2 et 3, caractérisés en ce qu'ils contiennent, comme polyisocyanate organique a), des polyisocyanates de faible poids moléculaire inférieur à 400, des produits de modification de tels polyisocyanates de faible poids moléculaire entre 400 et 10.000, qui peut être calculé à partir de la fonctionnalité et de la teneur en groupes fonctionnels ou encore des polyisocyanates à poids moléculaire élevé contenant, par exemple, des unités de structure isocyanurate, carbodiimide, allophanate, biuret ou uretdione.

5. Bandages de soutien selon les revendications 1 à 4, caractérisés en ce qu'ils contiennent, comme catalyseur b), des catalyseurs anioniques ou des polyéthers amorcés par des amines.

6. Bandages de soutien selon les revendications 1 à 5, caractérisés en ce qu'ils contiennent, comme matière de support, des feuilles pleines ou poreuses ou encore des mousses en matières naturelles ou synthétiques ou bien des produits de surface flexibles perméables à l'air à base de textile.

7. Procédé pour la préparation de bandages de soutien selon la revendication 1, caractérisé par la répartition homogène par-dessus la surface de la matière de support et en l'absence d'humidité, d'une préparation de polyisocyanate constituée par
   a) au moins un polyisocyanate organique,
   b) au moins un catalyseur et
   c) des adjuvants et des additifs de structure I selon la revendication 1.

8. Procédé pour la préparation de bandages de soutien selon la revendication 7, caractérisé en ce que la teneur en préparation de polyisocyanate s'élève de 25 à 90% en poids, par rapport au bandage de soutien total.

9. Procédé pour la préparation de bandages de soutien selon les revendications 7 et 8, caractérisé par la dissolution de la préparation de polyisocyanate dans un solvant inerte, la répartition homogéne de la solution par-dessus la surface de la matière de support et l'élimination du solvant.

10. Utilisation de préparations de polyisocyanates composées de
   a) au moins un polyisocyanate organique,
   b) au moins un catalyseur et
   c) des adjuvants et des additifs, dans lesquelles sont utilisés, comme adjuvant et additif selon l'invention, des composés de structure I selon la revendication 1,
   pour la préparation de bandages de soutien à des fins médicales ou techniques.

**Revendications pour l'Etat contractant suivant : ES**

1. Procédé pour la préparation de bandages de soutien qui contiennent, sur une matière de support flexible, une préparation de polyisocyanate constituée par :
   a) au moins un polyisocyanate organique,
   b) au moins un catalyseur et
   c) des adjuvant et des additifs, se caractérisant par le fait que les adjuvants et les additifs correspondent à des composés de structure I

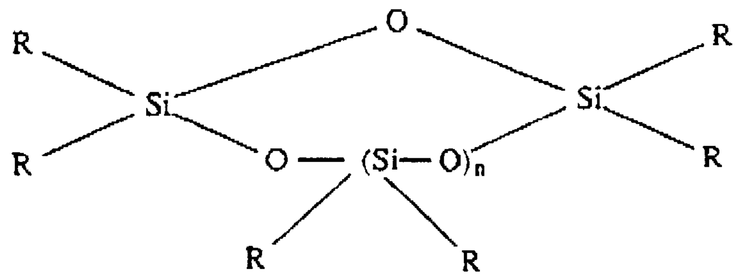

(I)

dans laquelle R représente un groupe méthyle, éthyle ou propyle et 1 ≦ n ≦ 4, caractérisé par la répartition homogène par-dessus la surface de la matière de support et en l'absence d'humidité, d'une préparation de polyisocyanate composée de

a) au moins un polyisocyanate organique,
b) au moins un catalyseur et
c) des adjuvants et des additifs de structure I.

2. Procédé selon la revendication 1, caractérisé en ce que la teneur en préparation de polyisocyanate s'élève de 25 à 90% en poids, par rapport au bandage de soutien total.

3. Procédé selon les revendications 1 et 2, caractérisé par la dissolution de la préparation de polyisocyanate dans un solvant inerte, la répartition homogène de la solution par-dessus la surface de la matière de support et l'élimination du solvant.